# EUROPEAN PATENT APPLICATION

(11) **EP 4 847 127 A1**
(43) Date of publication of application: **02.12.2026**
(21) Application number: 25745322.5
(22) Date of filing: 22.01.2025
(51) Int. Cl.: B01J 23/72, B01J 37/00, B01J 37/08, B01J 21/08, B01J 35/61, B01J 35/37, B01J 35/50, B01J 37/03

(54) **COPPER SILICATE-BASED CATALYST AND MANUFACTURING METHOD THEREOF**

(30) Priority: 23.01.2024 KR 20240009948
(71) Applicant: LG CHEM, LTD., Seoul 07336 (KR)
(72) Inventor: SUH, Myungji, Daejeon 34122 (KR); YANG, Sungpil, Daejeon 34122 (KR); EOM, Sungshik, Daejeon 34122 (KR)
(74) Representative: Goddar, Heinz J.
(86) International application number: PCT/KR2025/001236
(87) International publication number: WO 2025/159497

(57) **Abstract**

A copper silicate-based catalyst according to an exemplary embodiment of the present application is a copper silicate-based catalyst containing copper and silica, wherein the weight ratio of Cu : Si in the copper silicate-based catalyst is 30 : 70 to 70 : 30, the copper silicate-based catalyst has the content of Cu with an oxidation number of 2 of 2 at% to 6 at% upon XPS (X-ray photoelectron spectroscopy) analysis, and the copper silicate-based catalyst is in the form of a cylindrical pellet with a diameter of 3 mm to 6 mm and a height of 3 mm to 9 mm.

## Description

### [Technical Field]

This application claims the benefit of Korean Patent Application No. 10-2024-0009948 filed in the Korean Intellectual Property Office on January 23, 2024, the entire contents of which are incorporated herein by reference.

This application relates to a copper silicate-based catalyst and a method for preparing the same.

### [Background Art]

Neopentyl glycol (NPG) is a white crystalline substance with a melting point of 130 °C or higher. It is used as an important intermediate for various synthetic resins and is also widely used industrially as a raw material for various plastic powder paints, synthetic lubricants, plasticizers, surfactants, fiber processing agents, etc.

The NPG is generally prepared by preparing hydroxypivaldehyde (HPA) through an aldol condensation reaction of isobutyraldehyde and formaldehyde, and then reacting the HPA with hydrogen in the presence of a catalyst.

Conventionally, the HPA has been subjected to hydrogenation reaction using a slurry-type Ni-based catalyst. In this case, crude NPG, which is a hydrogenation reaction product, contains 2,2,4-trimethyl-1,3-pentanediol (TMPD), hydroxypivalic acid NPG ester (HPNE), etc. Since TMPD and HPNE have boiling points very similar to that of NPG, they cannot be separated by simple distillation. Since HPNE is unstable when distilling the reaction mixture and leads to a decrease in the yield of NPG, it is commercially converted to NPG by saponification reaction by adding sodium hydroxide. However, since sodium salts of HPA or other organic acids produced by the saponification reaction promote the decomposition reaction of NPG at high temperatures of 140 °C or higher, the distillation process is constrained. Furthermore, it is impossible to remove TMPD which has not been converted to a non-volatile sodium salt during the saponification reaction.

Therefore, efforts for producing NPG at a high yield in an economical way are being made continuously in the art.

### [Disclosure]

### [Technical Problem]

The present application provides a copper silicate-based catalyst and a method for preparing the same.

### [Technical Solution]

An exemplary embodiment of the present application provides a copper silicate-based catalyst containing copper and silica, wherein
a weight ratio of Cu : Si in the copper silicate-based catalyst is 30 : 70 to 70 : 30,
the copper silicate-based catalyst has a content of Cu with an oxidation number of 2 of 2 at% to 6 at% upon XPS (X-ray photoelectron spectroscopy) analysis, and
the copper silicate-based catalyst is in the form of a cylindrical pellet with a diameter of 3 mm to 6 mm and a height of 3 mm to 9 mm.

Another exemplary embodiment of the present application provides a method for preparing a copper silicate-based catalyst, which comprises:
a step of preparing a coprecipitated slurry by adding a silica sol and an alkaline precipitant to an aqueous solution containing a copper precursor;
a step of preparing a dried product by filtering and drying the coprecipitated slurry;
a step of mixing the dried product with a fiber-based binder and an organic compound, and then preparing a catalyst precursor by molding the same into a cylindrical pellet with a diameter of 3 mm to 6 mm and a height of 3 mm to 9 mm; and
a step of drying and sintering the catalyst precursor.

In addition, another exemplary embodiment of the present application provides a method for preparing neopentyl glycol, which comprises a step of conducting hydrogenation reaction by adding a hydroxypivaldehyde (HPA) solution and hydrogen to a hydrogenation reactor, wherein
the hydrogenation reactor contains the copper silicate-based catalyst.

### [Advantageous Effects]

According to an exemplary embodiment of the present application, a copper silicate-based catalyst with improved stability can be prepared. In particular, the copper silicate-based catalyst according to an exemplary embodiment of the present application, which is a catalyst molded into a cylindrical pellet with a diameter of 3 mm to 6 mm and a height of 3 mm to 9 mm by mixing with a fiber-based binder and an organic compound, is advantageous in that the content of Cu with an oxidation number of 2 and specific surface area characteristics of the catalyst can be optimized and, therefore, the decline in the strength, reactivity, etc. of the catalyst can be prevented even under the high-temperature, high-pressure reaction condition for preparation of neopentyl glycol with a high yield.

Accordingly, when the copper silicate-based catalyst according to an exemplary embodiment of the present application is used to prepare neopentyl glycol, the preparation process can be maintained stably and, therefore, economical efficiency can be enhanced.

### [Brief Description of Drawings]

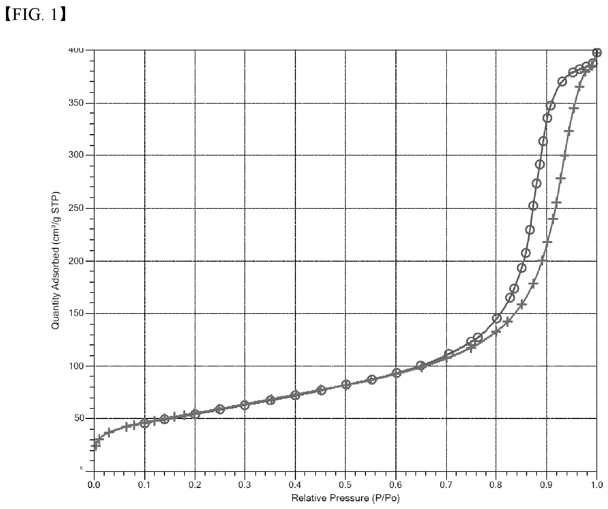

FIG. 1 shows nitrogen adsorption/desorption isotherms for measuring the BET surface area of a copper silicate-based catalyst of Example 1 according to the present application.

### [Best Mode]

Hereinafter, the present specification is described in more detail.

In the present specification, when a member is described to be located "on" another member, it comprises not only the case where the two members are in contact with each other but also the case where another member is present between the two members.

In the present specification, when a part is described to "comprise" a certain component, it means that it may comprise other components and does not exclude other components, unless the context specifically states otherwise.

As stated above, efforts for producing NPG at a high yield in an economical way are being made continuously in the art.

In particular, neopentyl glycol is prepared by hydrogenation of HPA (hydroxypivaldehyde) in the presence of a catalyst under high-temperature (160 °C or higher) and high-pressure (35 bar or higher) conditions. Since the strength and reactivity of the catalyst may decline under the high-temperature, high-pressure reaction conditions due to the elution of catalyst components, the hydrogenation process may not be operated stably.

Therefore, the present application is directed to providing a copper silicate-based catalyst with superior stability, which can be applied to the preparation process of neopentyl glycol, and a method for preparing the same.

The copper silicate-based catalyst according to an exemplary embodiment of the present application contains copper and silica, wherein the weight ratio of Cu : Si in the copper silicate-based catalyst is 30 : 70 to 70 : 30, the content of Cu with an oxidation number of 2 of the copper silicate-based catalyst upon XPS (X-ray photoelectron spectroscopy) analysis is 2 at% to 6 at%, and the copper silicate-based catalyst is in the form of a cylindrical pellet with a diameter of 3 mm to 6 mm and a height of 3 mm to 9 mm.

In an exemplary embodiment of the present application, the weight ratio of Cu : Si in the copper silicate-based catalyst may be 30 : 70 to 70 : 30, 30 : 70 to 50 : 50, or 35 : 65 to 50 : 50. The weight ratio of Cu : Si in the copper silicate-based catalyst is an indicator of the content of Cu, which is the active component of the catalyst. If the weight ratio of Cu : Si is outside the above ranges, the activity of the catalyst declines undesirably.

In an exemplary embodiment of the present application, the copper silicate-based catalyst may have the content of Cu with an oxidation number of 2 of 2 at% to 6 at%, 2.1 at% to 5 at%, or 2.2 at% to 3.0 at%, upon XPS (X-ray photoelectron spectroscopy) analysis. When the content of Cu with an oxidation number of 2 of the copper silicate-based catalyst upon XPS (X-ray photoelectron spectroscopy) analysis satisfies the above ranges, superior catalytic activity may be achieved.

A specific method for measuring the Cu with an oxidation number of 2 of the copper silicate-based catalyst by XPS (X-ray photoelectron spectroscopy) analysis is described in the examples given below.

In an exemplary embodiment of the present application, the copper silicate-based catalyst may be in the form of a cylindrical pellet with a diameter of 3 mm to 6 mm and a height of 3 mm to 9 mm, a cylindrical pellet with a diameter of 3.5 mm to 5 mm and a height of 4 mm to 8 mm, or a cylindrical pellet with a diameter of 5 mm and a height of 5 mm. If the diameter of the cylindrical pellet exceeds 6 mm or if the height exceeds 9 mm, catalytic activity may decline undesirably due to decreased surface area. In addition, if the diameter of the cylindrical pellet is smaller than 3 mm or if the height is smaller than 3 mm, crushing and drifting may occur undesirably under the high-temperature, high-pressure reaction condition for preparation of neopentyl glycol.

The molding of the catalyst is important for physical durability and flow of reactants in the reactor. Although a powder catalyst may be advantageous in terms of catalytic activity because of large surface area due to small particle size, the flow of reactants in a commercial-scale fixed-bed reactor is limited and drifting or differential pressure may occur. For this reason, it is desirable to mold the catalyst by extrusion, compression, coating, etc. The pores formed by the molded catalyst in the fixed-bed reactor provide the effect of reducing differential pressure caused by reactants supplied at a high flow rate for commercial productivity. In particular, since the extrusion-molded catalyst has the advantage of high durability due to high strength, the possibility of drifting or differential pressure caused by wearing, crushing, etc. during long-term use of the catalyst may be decreased.

In an exemplary embodiment of the present application, the copper silicate-based catalyst may have a specific surface area of 1 m²/g to 200 m²/g, 20 m²/g to 180 m²/g, or 64 m²/g to 128 m²/g. If the specific surface area of the copper silicate-based catalyst is outside the above ranges, catalytic activity may decline undesirably.

In an exemplary embodiment of the present application, the copper silicate-based catalyst may have a Si elution ratio of 5 wt% or lower, 3 wt% or lower, or 0, after an accelerated test according to Method 1:

### [Method 1]

After immersing the copper silicate-based catalyst in a hydroxypivaldehyde (HPA) solution and conducting reaction at 180 °C for 6 hours under hydrogen atmosphere, the elution ratio of Si is calculated according to Equation 2. [Equation 2] 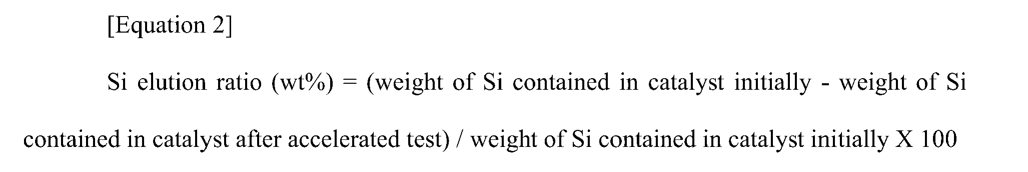 Si elution ratio (wt%) = (weight of Si contained in catalyst initially - weight of Si contained in catalyst after accelerated test) / weight of Si contained in catalyst initially X 100

If the Si elution ratio of the copper silicate-based catalyst exceeds 5 wt% after an accelerated test according to Method 1, the strength and reactivity of the catalyst may decline due to elution of Si during a preparation process of neopentyl glycol by hydrogenation of HPA under high-temperature, high-pressure reaction conditions. This may cause problems in stable operation of the hydrogenation process.

In an exemplary embodiment of the present application, the copper silicate-based catalyst may have a crushing strength decline ratio of 10% or lower, 5% or lower, 3% or lower, or 0, after an accelerated test according to Method 2.

### [Method 2]

After immersing the copper silicate-based catalyst in a hydroxypivaldehyde (HPA) solution and conducting reaction at 180 °C for 6 hours under hydrogen atmosphere, the crushing strength decline ratio is calculated according to Equation 3. [Equation 3] 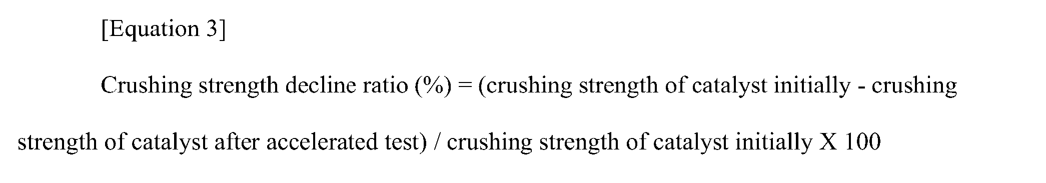 Crushing strength decline ratio (%) = (crushing strength of catalyst initially - crushing strength of catalyst after accelerated test) / crushing strength of catalyst initially X 100

If the crushing strength decline ratio of the copper silicate-based catalyst exceeds 10% after an accelerated test according to Method 2, the strength and reactivity of the catalyst may decline due to elution of Si during a preparation process of neopentyl glycol by hydrogenation of HPA under high-temperature, high-pressure reaction conditions. This may cause problems in stable operation of the hydrogenation process.

A specific method for measuring the specific surface area of the copper silicate-based catalyst is described in the examples given below.

In an exemplary embodiment of the present application, the copper silicate-based catalyst may have a crushing strength of 40 N or higher, specifically 42 N to 150 N, or 52 N to 89 N. If the crushing strength of the copper silicate-based catalyst is lower than 40 N, the catalyst may be damaged easily when it is put in a reactor, and a differential pressure may be formed undesirably in the reactor due to damage within a relatively short period.

A specific method for measuring the crushing strength of the copper silicate-based catalyst is described in the examples given below.

In an exemplary embodiment of the present application, the copper silicate-based catalyst may be for preparation of neopentyl glycol.

In addition, a method for preparing a copper silicate-based catalyst according to an exemplary embodiment of the present application comprises: a step of preparing a coprecipitated slurry by adding a silica sol and an alkaline precipitant to an aqueous solution containing a copper precursor; a step of preparing a dried product by filtering and drying the coprecipitated slurry; a step of mixing the dried product with a fiber-based binder and an organic compound, and then preparing a catalyst precursor by molding the same into a cylindrical pellet with a diameter of 3 mm to 6 mm and a height of 3 mm to 9 mm; and a step of drying and sintering the catalyst precursor.

The method for preparing a copper silicate-based catalyst according to an exemplary embodiment of the present application comprises a step of preparing a coprecipitated slurry by adding a silica sol and an alkaline precipitant to an aqueous solution containing a copper precursor.

In an exemplary embodiment of the present application, the copper precursor may be Cu(NO₃)₂·3H₂O, Cu(CO₃)₂·Cu(OH)₂, CuCl₂·2H₂O, etc., although not being limited thereto.

In an exemplary embodiment of the present application, the alkaline precipitant may be an alkali metal hydroxide, an alkali metal carbonate, an alkali metal bicarbonate, a mixture thereof, etc. More specifically, the alkaline precipitant may be one or more of NH₄OH, (NH₄)₂CO₃, NH₄HCO₃, CH₄N₂O, NaOH and Na₂CO₃, although not being limited thereto.

When adding the alkaline precipitant, the temperature increased by heat of neutralization may be lowered to room temperature using cooling water, etc.

The method for preparing a copper silicate-based catalyst according to an exemplary embodiment of the present application may further comprise a step of aging the coprecipitated slurry at 80 °C to 90 °C for 3 hours or longer. The aging may be performed at 80 °C to 90 °C for 3 hours or longer, or at 80 °C to 90 °C for 3 hours to 8 hours.

Bonding between the copper precursor and the silica is formed by the aging. The copper precursor does not remain unreacted when both the aging temperature and time described above are satisfied. If the copper precursor remains unreacted, the activity of the catalyst may decrease undesirably.

The method for preparing a copper silicate-based catalyst according to an exemplary embodiment of the present application comprises a step of preparing a dried product by filtering and drying the coprecipitated slurry.

The coprecipitated slurry may be filtered by a method known in the art without special limitation.

In an exemplary embodiment of the present application, the filtered coprecipitated slurry may be dried at 60 °C to 120 °C for 5 hours to 72 hours, or at 70 °C to 110 °C for 7 hours to 48 hours.

The method for preparing a copper silicate-based catalyst according to an exemplary embodiment of the present application comprises a step of mixing the dried product with a fiber-based binder and an organic compound, and then preparing a catalyst precursor by molding the same into a cylindrical pellet with a diameter of 3 mm to 6 mm and a height of 3 mm to 9 mm.

In an exemplary embodiment of the present application, the fiber-based binder may comprise one or more of glass fiber, carbon fiber, aramid fiber, alumina fiber, aluminum silicate fiber, silicon carbide fiber and boron fiber.

In an exemplary embodiment of the present application, the content of the fiber-based binder may be 0.1 wt% or more and less than 15 wt%, 1 wt% to 10 wt%, or 3 wt% to 7 wt%, based on the total weight of the dried product. If the content of the fiber-based binder is less than 0.1 wt% based on the total weight of the dried product, physical stability may worsen undesirably under the high-temperature, high-pressure reaction condition due to significantly decreased strength of the catalyst. And, if the content of the fiber-based binder is 15 wt% or more, productivity may decrease during the preparation of the catalyst because of poor flowability during the extrusion molding of the catalyst and the strength of the catalyst may also decline because of poor compressibility.

In an exemplary embodiment of the present application, the content of the organic compound may be 0.1 wt% to 50 wt%, 1 wt% to 40 wt%, or 20 wt% to 30 wt%, based on the total weight of the dried product. The organic compound serves as a lubricant during the molding of the catalyst. If the content of the organic compound is less than 0.1 wt% based on the total weight of the dried product, molding may be impossible. And, if it exceeds 50 wt%, the strength or activity of the catalyst may decline undesirably.

In an exemplary embodiment of the present application, the organic compound may comprise one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

In an exemplary embodiment of the present application, after the dried product is mixed with a fiber-based binder and an organic compound, a catalyst precursor in the form of a cylindrical pellet with a diameter of 3 mm to 6 mm and a height of 3 mm to 9 mm, or a catalyst precursor in the form of a cylindrical pellet with a diameter of 3.5 mm to 5 mm and a height of 4 mm to 8 mm may be prepared by molding the same. If the diameter of the cylindrical pellet exceeds 6 mm or if the height exceeds 9 mm, catalytic activity may decline undesirably due to decreased surface area. In addition, if the diameter of the cylindrical pellet is smaller than 3 mm or if the height is smaller than 3 mm, crushing and drifting may occur undesirably under the high-temperature, high-pressure reaction condition for preparation of neopentyl glycol.

The method for preparing a copper silicate-based catalyst according to an exemplary embodiment of the present application comprises a step of drying and sintering the catalyst precursor.

In an exemplary embodiment of the present application, the catalyst precursor may be dried at 60 °C to 120 °C for 5 hours to 24 hours, or at 70 °C to 110 °C for 7 hours to 20 hours.

In an exemplary embodiment of the present application, the dried catalyst precursor may be sintered at 350 °C to 600 °C for 5 hours to 24 hours, or at 400 °C to 550 °C for 7 hours to 20 hours.

If the drying condition and sintering condition are outside the above ranges, catalytic activity may decline undesirably due to change in the crystallinity of the catalyst.

In addition, another exemplary embodiment of the present application provides a method for preparing neopentyl glycol, which comprises a step of adding a hydroxypivaldehyde (HPA) solution and hydrogen to a hydrogenation reactor and conducting hydrogenation reaction, wherein the hydrogenation reactor contains the copper silicate-based catalyst.

In an exemplary embodiment of the present application, a method known in the art may be used for the preparation of neopentyl glycol, except for using the copper silicate-based catalyst.

For example, the hydrogenation reactor may be a fixed-bed reactor (FBR) charged with the copper silicate-based catalyst. In this case, it is not necessary to separate the reaction product from the catalyst, the operation is stable and economical because reaction temperature and reaction pressure can be lowered, the replacement of the catalyst is easy, and the investment cost can be reduced greatly because the reactor size can be decreased.

In addition, the hydroxypivaldehyde solution may contain 65 wt% or less of hydroxypivaldehyde, 10 wt% or less of neopentyl glycol, 15 wt% to 35 wt% of an alcohol, and 15 wt% or less of water. In this case, the production of byproducts may be suppressed since the heat of reaction can be minimized without sacrificing reactivity.

The hydrogenation reaction may be performed at 100 °C to 250 °C, 100 °C to 200 °C, or 100 °C to 180 °C.

According to an exemplary embodiment of the present application, a copper silicate-based catalyst with improved stability may be prepared. In particular, the copper silicate-based catalyst according to an exemplary embodiment of the present application, which is prepared by molding after mixing with a fiber-based binder and an organic compound, may have an optimized content of Cu with an oxidation number of 2 and specific surface area. As a result, the decline in the strength, reactivity, etc. of the catalyst may be prevented even under the high-temperature, high-pressure reaction condition for preparation of neopentyl glycol with a high yield.

Accordingly, when the copper silicate-based catalyst according to an exemplary embodiment of the present application is used to prepare neopentyl glycol, the preparation process can be maintained stably and, therefore, economical efficiency can be enhanced.

### [Mode for Invention]

Hereinafter, the present application will be described in detail through specific examples. However, the examples according to the present application can be changed into various other forms, and it should not be interpreted that the scope of the present application is limited by the examples described below. The examples of the present application are provided to describe the present application more completely to those having ordinary knowledge in the art.

### <Examples>

### <Example 1>

A copper precursor (Cu(NO₃)₂·3H₂O) aqueous solution was prepared in a double jacket reactor, and silica sol was added such that the weight ratio of Cu and Si was Cu : Si = 30 : 70. After adding a NaOH aqueous solution, a coprecipitate was prepared by increasing the temperature of the reactor and performing aging at 80 °C for 3 hours. After filtering the coprecipitate and washing with distilled water, a wet cake obtained thereby was dried in an oven. The dried product was crushed to obtain a catalyst intermediate powder.

After mixing 25 wt% of an organic compound (20 wt% of isopropyl alcohol and 5 wt% of glycerin), 5 wt% of a fiber-based binder (ceramic wool, aluminum silicate) and distilled water, based on the total weight of the catalyst intermediate powder, the catalyst intermediate was extrusion-molded into a cylindrical pellet with a diameter of 5 mm and a length of 5 mm using an extruder.

Finally, a catalyst for preparation of neopentyl glycol was prepared by sintering the extrusion-molded catalyst intermediate at 550 °C for 8 hours.

### <Example 2>

A catalyst for preparation of neopentyl glycol was prepared in the same manner as in Example 1, except that the coprecipitate was prepared such that the weight ratio of Cu and Si was 50 : 50.

### <Example 3>

A catalyst for preparation of neopentyl glycol was prepared in the same manner as in Example 1, except that 10 wt% of the fiber-based binder was used in the extrusion molding step, instead of 5 wt% of the fiber-based binder.

### <Comparative Example 1>

A catalyst for preparation of neopentyl glycol was prepared in the same manner as in Example 1, except that a cylindrical pellet with a diameter 2.5 mm and a length of 10 mm, rather than a diameter 5 mm and a length of 5 mm, was extrusion-molded in the extrusion molding step.

### <Comparative Example 2>

A catalyst for preparation of neopentyl glycol was prepared in the same manner as in Example 1, except that the fiber-based binder was not used in the extrusion molding step.

### <Comparative Example 3>

A catalyst for preparation of neopentyl glycol was prepared in the same manner as in Example 1, except that the organic compound was not used in the extrusion molding step. However, extrusion was impossible because lubrication was not performed smoothly in the extruder during the extrusion molding step. As a result, a catalyst could not be prepared.

### <Test Example>

In the presence of the catalyst prepared in the examples or comparative examples, hydrogenation reaction was performed at 160 °C and 35 bar for 1 hour using an HPA solution consisting of 65 wt% of HPA, 2 wt% of NPG, 25 wt% of 2-ethylhexanol, 5 wt% of H₂O and 3 wt% of a high-boiling-point material, and catalytic activity was measured by measuring the amount of hydrogen consumed. The result is shown in Table 1. The catalytic activity was represented relative to the result of Example 1 as 100.

In addition, the crushing strength and BET surface area of the catalysts of the examples and comparative examples were evaluated by nitrogen adsorption/desorption experiment. The result is shown in Table 1.

In addition, the Cu(II) content of the catalysts of the examples and comparative examples upon XPS (X-ray photoelectron spectroscopy) analysis, and the Si elution ratio and strength decline ratio after an accelerated test were evaluated. The result is shown in Table 2.

FIG. 1 shows the nitrogen adsorption/desorption isotherms for measuring the BET surface area of the copper silicate-based catalyst of Example 1.

The measurement methods of the evaluation results shown in Tables 1 and 2 are as follows.

### <Crushing strength>

Crushing strength was measured using SHIMPO's FGN-50B. More specifically, after placing the catalyst extrusion-molded to a cylindrical shape on a strength tester holder such that the side surface faces the bottom, the maximum pressure value was measured as the measuring unit compresses the catalyst downward from the top. Measurement was made for 20 catalysts and the result was averaged.

For Example 1, although it was intended to measure the crushing strength of the catalyst intermediate powder without conducting the extrusion molding step, the measurement was impossible with the method using SHIMPO's FGN-50B because the particle size of the powder catalyst was small as about 100 µm. Thus, the crushing strength of the powder catalyst was evaluated as 0.

### <BET surface area>

BET surface area was calculated from the BET (Brunauer-Emmett-Teller) equation by measuring the amount of adsorbed/desorbed nitrogen depending on nitrogen partial pressure.

### <Cu(II) content>

Cu(II) content was measured by XPS (X-ray photoelectron spectroscopy) analysis.

More specifically, after preparing a circular pellet sample with a diameter of 2 mm and a thickness of 1 mm by crushing the catalyst, the sample was measured using a monochromatic Al k-alpha X-ray α (1486.6 eV) with a size of 400 µm X 800 µm. After obtaining a first XPS spectrum (before correction) by XPS (X-ray photoelectron spectroscopy) under the condition of vacuum atmosphere, a measurement range of 925 eV to 970 eV, and a pass energy of 50 eV, a second XPS spectrum (after correction) was obtained by correcting the peaks of the XPS spectrum. The Cu(II) content refers to the relative proportion of Cu with an oxidation number of 2 derived from the second XPS spectrum.

### <Accelerated test>

After immersing the catalyst of the examples or comparative examples in a HPA solution, accelerated test was performed by conducting reaction at 180 °C for 6 hours under hydrogen atmosphere. Then, the solution remaining on the catalyst was removed by washing with acetone and water and then drying at room temperature. The elution ratio of Si was calculated according to Equation 1. In addition, the strength decline ratio of the catalyst was calculated according to Equation 2. [Equation 1] 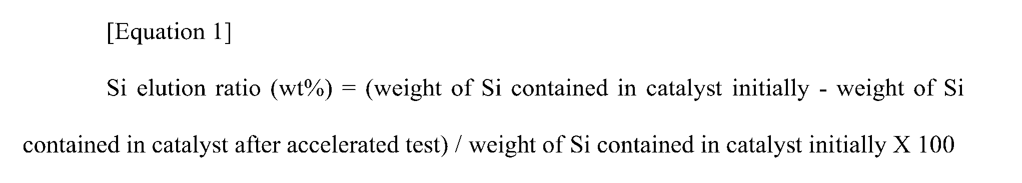 Si elution ratio (wt%) = (weight of Si contained in catalyst initially - weight of Si contained in catalyst after accelerated test) / weight of Si contained in catalyst initially X 100 [Equation 2] 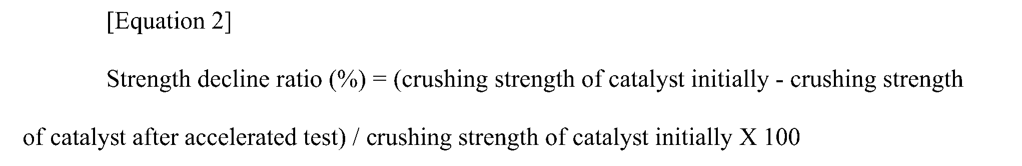 Strength decline ratio (%) = (crushing strength of catalyst initially - crushing strength of catalyst after accelerated test) / crushing strength of catalyst initially X 100

**[Table 1]**

| | Catalytic activity | Crushing strength (N) | BET surface area (m²/g) |
|---|---|---|---|
| Example 1 | 100 | 65 | 128 |
| Example 2 | 106 | 89 | 93 |
| Example 3 | 111 | 52 | 64 |
| Comparative Example 1 | 99 | 23 | 157 |
| Comparative Example 2 | 57 | 27 | 18 |

**[Table 2]**

| | XPS | Accelerated test | |
|---|---|---|---|
| | Cu(II) content (at%) | Si elution ratio (%) | Strength decline ratio (%) |
| Example 1 | 3.0 | 2 | 1 |
| Example 2 | 2.8 | 3 | 0 |
| Example 3 | 2.2 | 0 | 0 |
| Comparative Example 1 | 6.2 | 11 | 25 |
| Comparative Example 2 | 7.5 | 19 | 28 |

As shown in Tables 1 and 2, the catalysts for preparation of neopentyl glycol of Examples 1 to 3 exhibited superior catalytic activity as compared to the catalysts of Comparative Examples 1 and 2, with the content of Cu with an oxidation number of 2 determined by XPS (X-ray photoelectron spectroscopy) analysis in the range of 2 at% to 6 at%.

The catalyst for preparation of neopentyl glycol of Comparative Example 1, which was prepared into a cylindrical pellet with a smaller size than those of the examples, exhibited weak catalyst strength and low physical stability due to easy elution of Si. In addition, the catalyst of Comparative Example 2, wherein the fiber-based binder was not used, showed declined catalyst strength and catalytic activity.

In addition, the catalyst for preparation of neopentyl glycol according to an exemplary embodiment of the present application, which is a catalyst extrusion-molded into a cylindrical shape, is applicable to a fixed-bed reactor of a commercial scale, to which an unmolded powder catalyst is difficult to be applied, and the possibility of drifting or differential pressure caused by wearing, crushing, etc. during long-term use of the catalyst may be decreased.

Accordingly, since the catalyst for preparation of neopentyl glycol according to an exemplary embodiment of the present application can maintain catalytic activity even under high-temperature, high-pressure reaction conditions due to superior strength, reactivity and stability of the catalyst, it can improve the stability of a process for preparing neopentyl glycol with superior catalytic activity.

## Claims

1. A copper silicate-based catalyst comprising copper and silica, wherein
a weight ratio of Cu : Si in the copper silicate-based catalyst is 30 : 70 to 70 : 30,
a content of Cu with an oxidation number of 2 of the copper silicate-based catalyst upon XPS (X-ray photoelectron spectroscopy) analysis is of 2 at% to 6 at%, and
the copper silicate-based catalyst is in the form of a cylindrical pellet with a diameter of 3 mm to 6 mm and a height of 3 mm to 9 mm.

2. The copper silicate-based catalyst according to claim 1, wherein the copper silicate-based catalyst has a specific surface area of 1 m²/g to 200 m²/g.

3. The copper silicate-based catalyst according to claim 1, wherein the copper silicate-based catalyst has a crushing strength of 40 N or higher.

4. The copper silicate-based catalyst according to claim 1, wherein the copper silicate-based catalyst has a Si elution ratio of 5 wt% or lower after an accelerated test according to Method 1:
[Method 1]
after immersing the copper silicate-based catalyst in a hydroxypivaldehyde (HPA) solution and conducting reaction at 180 °C for 6 hours under hydrogen atmosphere, the elution ratio of Si is calculated according to Equation 2: [Equation 2] 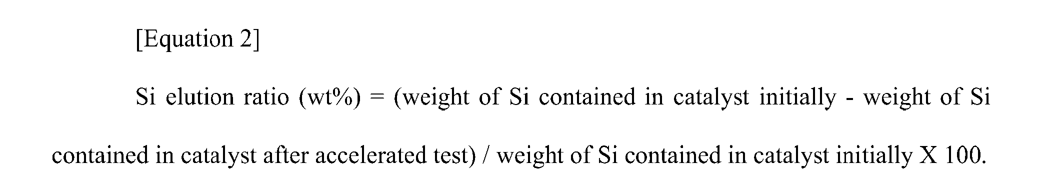 Si elution ratio (wt%) = (weight of Si contained in catalyst initially - weight of Si contained in catalyst after accelerated test) / weight of Si contained in catalyst initially X 100.

5. The copper silicate-based catalyst according to claim 1, wherein the copper silicate-based catalyst has a crushing strength decline ratio of 10% or lower after an accelerated test according to Method 2:
[Method 2]
after immersing the copper silicate-based catalyst in a hydroxypivaldehyde (HPA) solution and conducting reaction at 180 °C for 6 hours under hydrogen atmosphere, the crushing strength decline ratio is calculated according to Equation 3: [Equation 3] 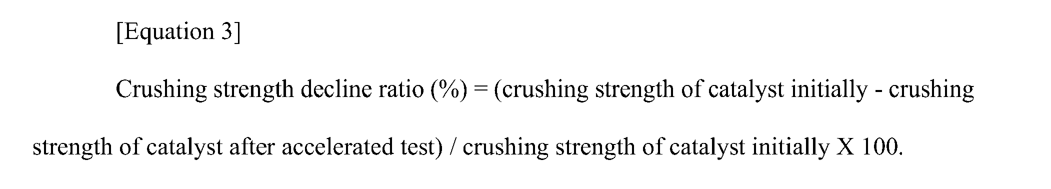 Crushing strength decline ratio (%) = (crushing strength of catalyst initially - crushing strength of catalyst after accelerated test) / crushing strength of catalyst initially X 100.

6. The copper silicate-based catalyst according to claim 1, wherein the copper silicate-based catalyst is for preparation of neopentyl glycol.

7. A method for preparing the copper silicate-based catalyst according to any of claims 1 to 6, comprising:
a step of preparing a coprecipitated slurry by adding a silica sol and an alkaline precipitant to an aqueous solution containing a copper precursor;
a step of preparing a dried product by filtering and drying the coprecipitated slurry;
a step of mixing the dried product with a fiber-based binder and an organic compound, and then preparing a catalyst precursor by molding the same into a cylindrical pellet with a diameter of 3 mm to 6 mm and a height of 3 mm to 9 mm; and
a step of drying and sintering the catalyst precursor.

8. The method for preparing the copper silicate-based catalyst according to claim 7, wherein the alkaline precipitant comprises one or more of NH₄OH, (NH₄)₂CO₃, NH₄HCO₃, CH₄N₂O, NaOH and Na₂CO₃.

9. The method for preparing the copper silicate-based catalyst according to claim 7, wherein the fiber-based binder comprises one or more of glass fiber, carbon fiber, aramid fiber, alumina fiber, aluminum silicate fiber, silicon carbide fiber and boron fiber.

10. The method for preparing the copper silicate-based catalyst according to claim 7, wherein a content of the fiber-based binder is 0.1 wt% or more and less than 15 wt% based on a total weight of the dried product.

11. The method for preparing the copper silicate-based catalyst according to claim 7, wherein the organic compound comprises one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

12. The method for preparing the copper silicate-based catalyst according to claim 7, wherein a content of the organic compound is 0.1 wt% to 50 wt% based on a total weight of the dried product.

13. A method for preparing neopentyl glycol, comprising a step of conducting hydrogenation reaction by adding a hydroxypivaldehyde (HPA) solution and hydrogen to a hydrogenation reactor, wherein
the hydrogenation reactor comprises the copper silicate-based catalyst according to any of claims 1 to 6.
